# EUROPEAN PATENT SPECIFICATION

(11) **EP 2 654 693 B1**
(45) Date of publication and mention of the grant of the patent: **25.01.2017**
(21) Application number: 11799678.5
(22) Date of filing: 15.12.2011
(51) Int. Cl.: A61K 8/365, A61K 8/73, A61Q 19/00, A61K 8/06

(54) **COSMETIC COMPOSITION COMPRISING A CUCURBIC ACID COMPOUND AND A HYDROPHOBIC INULIN**
KOSMETISCHE ZUSAMMENSETZUNG MIT EINER CUCURBINSÄUREVERBINDUNG UND EINEM HYDROPHOBEN INULIN
COMPOSITION COSMÉTIQUE COMPRENANT UN COMPOSÉ D'ACIDE CUCURBIQUE ET UNE INULINE HYDROPHOBE

(30) Priority: 20.12.2010 FR 1060829; 27.12.2010 US 201061427235 P
(43) Date of publication of application: 30.10.2013
(73) Proprietor: L'Oréal, 75008 Paris (FR)
(72) Inventor: CHARBIT, Yael, F-94400 Vitry Sur Seine (FR)
(74) Representative: Hugodot, Yannick
(86) International application number: PCT/EP2011/072982
(87) International publication number: WO 2012/084701

(56) References cited:
- EP-A2- 1 333 021
- WO-A1-99/64549
- FR-A1- 2 921 255
- BOOTEN K ET AL: "Polymeric, carbohydrate-based surfactants and their use in personal care applications", SOFW-JOURNAL SEIFEN, OELE, FETTE, WACHSE, VERLAG FUR CHEMISCHE INDUSTRIE, AUGSBURG, DE, vol. 130, no. 8, 1 August 2004 (2004-08-01), pages 10,12-16, XP001538872, ISSN: 0942-7694
- BOOTEN KARL ET AL: "Nature-based emulsifiers and their cosmetic applications", HAPPI HOUSEHOLD AND PERSONAL PRODUCTS INDUSTRY, RODMAN PUBLISHING, RAMSEY, NJ, US, vol. 41, no. 3, 1 March 2004 (2004-03-01), pages 66-67, XP009132712, ISSN: 0090-8878

## Description

The present invention relates to cosmetic compositions in oil-in-water emulsion form comprising a cucurbic acid compound and an inulin bearing hydrophobic groups, and also to the use of these compositions in a process for treating human keratin materials.
More particularly, the compositions of the invention are intended for caring for and/or making up keratin materials.

For the purposes of the invention, the term "keratin materials" is intended to denote, for example, the skin, mucous membranes, the lips, the scalp, the eyelashes, the eyebrows and the hair.

Patent application EP-A-1 333 021 discloses hydrogenated cucurbic acid compounds such as 3-hydroxy-2-pentylcyclopentaneacetic acid for promoting desquamation of the skin and stimulating epidermal renewal, combating the signs of ageing of the skin, improving the radiance of the complexion and/or making facial skin smooth. In patent application FR-A-62921255, these compounds are also described for their use as depigmenting agents.

However, introducing these hydrogenated cucurbic acid compounds into an oil-in-water emulsion gives rise to instability of the composition, especially after storage for one month, or even two months at room temperature (25°C): the emulsion then shows phase separation of oil at the surface; the oil globules dispersed in the aqueous phase have a coarse aspect, making the emulsion non-uniform.

The aim of the present invention is thus to provide an oil-in-water emulsion comprising the hydrogenated cucurbic acid compound that is stable, especially after storage for one month, or even two months at room temperature (25°C).

The inventor has discovered that such a stable emulsion can be obtained by using a particular inulin bearing hydrophobic groups.

More specifically, the present invention relates to a composition in oil-in-water emulsion form, comprising a cucurbic acid compound of formula (I), an inulin bearing a hydrophobic group chosen from hydrophobic carbamate or ester groups, an oily phase and an aqueous phase.

The composition according to the invention is in particular a cosmetic composition.

Surprisingly, the inventors have observed that the addition of a cucurbic acid compound to a composition comprising an inulin bearing a hydrophobic carbamate or ester group does not significantly affect the viscosity of the said composition and thus enables it to be formulated in a form that is suited to its handling during its application.

Further, the composition according to the invention presents good cosmetic properties such as for example, it is less sticky, easily spread (without drag) and bring comfort.

According to yet another of its subjects, the present invention relates to a non-therapeutic treatment process for caring for or making up keratin materials, comprising the application to the said keratin materials of a composition in accordance with the invention.

The cucurbic acid-based compound is a compound chosen from those corresponding to formula (I) below: 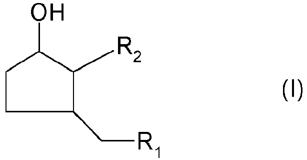 in which:
R₁ represents a radical COOR₃, R₃ denoting a hydrogen atom or a C₁-C₄ alkyl radical, optionally substituted with one or more hydroxyl groups;
- R₂ represents a saturated or unsaturated linear hydrocarbon-based radical containing from 1 to 18 carbon atoms or a saturated or unsaturated branched or cyclic hydrocarbon-based radical containing from 3 to 18 carbon atoms;
and also the optical isomers thereof, and the corresponding salts.

Preferably, R₁ denotes a radical chosen from -COOH, -COOMe, -COO-CH₂-CH₃, -COO-CH₂-CH(OH)-CH₂OH, -COOCH₂-CH₂-CH₂OH and -COOCH₂-CH(OH)-CH₃.
Preferentially, R₁ denotes a radical -COOH.

Preferentially, R₂ denotes a linear, saturated or unsaturated hydrocarbon-based radical, preferably containing from 2 to 7 carbon atoms. In particular, R₂ may be a pentyl, pentenyl, hexyl or heptyl radical.

According to one embodiment, the compound of formula (I) is chosen from 3-hydroxy-2-[(2Z)-2-pentenyl]cyclopentaneacetic acid and 3-hydroxy-2-pentylcyclopentaneacetic acid. Preferably, compound (I) is 3-hydroxy-2-pentylcyclopentaneacetic acid; this compound may especially be in the form of the sodium salt.

The salts of the compounds that may be used according to the invention are chosen in particular from salts of alkali metals, for example sodium or potassium; salts of alkaline-earth metals, for example calcium, magnesium or strontium, metal salts aluminium, manganese or copper; ammonium salts of formula NH₄⁺; quaternary ammonium salts; salts of organic amines, for instance salts of methylamine, dimethylamine, trimethylamine, triethylamine, ethylamine, 2-hydroxyethylamine, bis(2-hydroxyethyl)amine or tris(2-hydroxyethyl)amine; lysine or arginine salts. Salts chosen from sodium, potassium, magnesium, strontium, copper, manganese and zinc salts are preferably used. The sodium salt is preferentially used.

The compound of formula (I) defined previously may be present in the composition according to the invention in a content ranging from 1% to 10% by weight, and preferably from 1.5% to 5% by weight, relative to the total weight of the composition.

The composition according to the invention comprises an inulin bearing hydrophobic groups chosen from hydrophobic carbamate or ester groups.

The term "hydrophobic carbamate group" means a C₄-C₃₂ alkyl carbamate group, i.e. a group -OCONH-R, R being a C₄-C₃₂ alkyl.
The term "hydrophobic ester group" means a C₄-C₃₂ alkyl ester group, i.e. a group -OCO-R, R being a C₄-C₃₂ alkyl.

These hydrophobic groups are especially derived from the reaction of the hydroxyl groups of the starting inulin with either an isocyanate R-N=C=O (to form a carbamate group) or an acid R-COOH or acid chloride R-COCl (to form an ester group).

Inulin is part of the fructan family.
Fructans or fructosans are oligosaccharides or polysaccharides comprising a sequence of anhydrofructose units optionally combined with several saccharide residues other than fructose. Fructans may be linear or branched. Fructans may be products obtained directly from a plant or microbial source or alternatively products whose chain length has been modified (increased or decrease) by fractionation, synthesis or hydrolysis, in particular enzymatic. Fructans generally have a degree of polymerization from 2 to about 1000 and preferably from 2 to about 60.
Three groups of fructans are distinguished. The first group corresponds to products whose fructose units are for the most part linked via β-1-2 bonds. These are essentially linear fructans such as inulins.

The second group also corresponds to linear fructoses, but the fructose units are essentially linked via β-2-6 bonds. These products are levans.
The third group corresponds to mixed fructans, i.e. containing β-2-6 and β-2-1 sequences. These are essentially branched fructans, such as graminans.

Inulin may be obtained, for example, from chicory, dahlia or Jerusalem artichoke. Preferably, the inulin used in the composition according to the invention is obtained, for example, from chicory.

In particular, the inulin has a degree of polymerization that may range from 2 to 100 and preferably from 2 to 70.

Advantageously, the hydrophobic carbamate group is a C₆-C₂₀ alkyl carbamate group. Preferably, the hydrophobic carbamate group is a C₈-C₁₈ alkyl carbamate group. Preferentially, the hydrophobic carbamate group is a C₁₀-C₁₈ alkyl carbamate group. More preferentially, the hydrophobic carbamate group is a C₁₀-C₁₄ alkyl carbamate group.
According to a more preferred embodiment, the hydrophobic carbamate group is a lauryl carbamate group (C₁₂ alkyl group).

Inulins bearing hydrophobic carbamate groups are described, for example, in patent application WO 99/64549.

Advantageously, the hydrophobic ester group is a C₆-C₂₀ alkyl ester group. Preferably, the hydrophobic ester group is a C₈-C₂₀ alkyl ester group. Preferentially, the hydrophobic ester group is a C₁₀-C₂₀ alkyl ester group. More preferentially, the hydrophobic ester group is a C₁₀-C₁₈ alkyl ester group.

Inulins bearing hydrophobic ester groups are described, for example, in patent US 5 877 144.

Preferably, an inulin bearing hydrophobic carbamate groups is used.

The inulin bearing hydrophobic carbamate or ester groups may have a degree of substitution (proportion of OH of the inulin substituted with a hydrophobic group) ranging from 0.01 to 0.5, preferably ranging from 0.02 to 0.4 and preferentially ranging from 0.05 to 0.35. Advantageously, the degree of substitution may range from 0.1 to 0.3.

As examples of inulins bearing hydrophobic ester groups, mention may be made of stearoyl inulin, such as the products sold under the names Lifidrem INST by the company Engelhard and Rheopearl INS by the company Ciba; palmitoyl inulin; undecylenoyl inulin, such as the products sold under the names Lifidrem INUK and Lifidrem INUM by the company Engelhard.

An example of an inulin bearing hydrophobic carbonate groups that may be mentioned is inulin lauryl carbamate, such as the product sold under the name Inutec SP1 by the company Beneo.

The inulin bearing hydrophobic ester or carbamate groups may be present in the composition according to the invention in a content ranging from 0.1% to 3% by weight relative to the total weight of the composition. Preferably, the content of the said copolymer may range from 0.1% to 2% by weight relative to the total weight of the composition.
Preferentially, the content of the said copolymer may range from 0.2% to 2.5% by weight relative to the total weight of the composition.

Advantageously, the cucurbic acid compound of formula (I) (referred to as A) and the inulin bearing hydrophobic groups (referred to as B) described previously may be present in the composition according to the invention in an A/B weight ratio ranging from 3 to 10.
Preferably, this A/B weight ratio may range from 4 to 9.

The viscosity of a composition of the invention may be measured via any process known to those skilled in the art, and especially according to the following conventional process. Thus, the measurement may be performed at 25°C using a Rheomat 180 viscometer equipped with a spindle rotating at 200 rpm. A person skilled in the art may select the spindle for measuring the viscosity from the spindles M1, M2, M3 and M4 on the basis of his general knowledge, so as to be able to perform the measurement.

The cosmetic composition according to the invention comprises a physiologically acceptable medium, i.e. a medium that is compatible with human keratin materials and/or fibres, for instance, in a non-limiting manner, the skin, mucous membranes, the nails, the scalp and the hair.

The composition comprises an aqueous phase.

The composition may comprise water in a content ranging from 20% to 95% by weight, preferably ranging from 30% to 90% by weight and preferentially ranging from 40% to 70% by weight, relative to the total weight of the composition.

The water may be a floral water such as cornflower water and/or a mineral water such as Vittel water, Lucas water or La Roche Posay water and/or a spring water.

The composition may also comprise an organic solvent that is water-miscible at room temperature (25°C), chosen especially from monoalcohols containing from 2 to 6 carbon atoms, such as ethanol or isopropanol;
polyols especially containing from 2 to 20 carbon atoms, preferably containing from 2 to 10 carbon atoms and preferentially containing from 2 to 6 carbon atoms, such as glycerol, propylene glycol, butylene glycol, pentylene glycol, hexylene glycol, dipropylene glycol or diethylene glycol;
glycol ethers (especially containing from 3 to 16 carbon atoms) such as mono-, di- or tripropylene glycol (C₁-C₄)alkyl ethers, and mono-, di- or triethylene glycol (C₁-C₄)alkyl ethers;
and mixtures thereof.

The composition according to the invention may comprise a solvent that is miscible with water at room temperature, in a content ranging from 1% to 20% by weight and preferably ranging from 3% to 15% by weight relative to the total weight of the composition.

Advantageously, the composition according to the invention has a pH ranging from 5.5 to 7.5.

The emulsion according to the invention also comprises an oily phase.

As oils that may be used in the composition of the invention, examples that may be mentioned include:
- hydrocarbon-based oils of plant origin, such as liquid triglycerides of fatty acids containing from 4 to 10 carbon atoms, for instance heptanoic or octanoic acid triglycerides, or alternatively, for example, sunflower oil, corn oil, soybean oil, marrow oil, grapeseed oil, sesame seed oil, hazelnut oil, apricot oil, macadamia oil, arara oil, castor oil, avocado oil, caprylic/capric acid triglycerides, for instance those sold by the company Stearineries Dubois or those sold under the names Miglyol 810, 812 and 818 by the company Dynamit Nobel, jojoba oil and shea butter oil;
- synthetic esters and ethers, especially of fatty acids, for instance the oils of formulae R₁COOR₂ and RiOR₂ in which R₁ represents a fatty acid residue containing from 8 to 29 carbon atoms and R₂ represents a branched or unbranched hydrocarbon-based chain containing from 3 to 30 carbon atoms, for instance Purcellin oil, isononyl isononanoate, isopropyl myristate, 2-ethylhexyl palmitate, 2-octyldodecyl stearate, 2-octyldodecyl erucate or isostearyl isostearate; hydroxylated esters, for instance isostearyl lactate, octyl hydroxystearate, octyldodecyl hydroxystearate, diisostearyl malate or triisocetyl citrate; fatty alcohol heptanoates, octanoates or decanoates; polyol esters, for instance propylene glycol dioctanoate, neopentyl glycol diheptanoate and diethylene glycol diisononanoate; and pentaerythritol esters, for instance pentaerythrityl tetraisostearate;

- linear or branched hydrocarbons of mineral or synthetic origin, such as volatile or non-volatile liquid paraffins, and derivatives thereof, petroleum jelly, polydecenes, and hydrogenated polyisobutene such as Parleam oil;
- fatty alcohols containing from 8 to 26 carbon atoms, for instance cetyl alcohol, stearyl alcohol and a mixture thereof (cetylstearyl alcohol), octyldodecanol, 2-butyloctanol, 2-hexyldecanol, 2-undecylpentadecanol, oleyl alcohol or linoleyl alcohol;
- partially hydrocarbon-based and/or silicone-based fluoro oils, for instance those described in document JP-A-2 295 912;
- silicone oils, for instance volatile or non-volatile polymethylsiloxanes (PDMS) with a linear or cyclic silicone chain, which are liquid or pasty at room temperature, especially cyclopolydimethylsiloxanes (cyclomethicones) such as cyclohexasiloxane; polydimethylsiloxanes comprising alkyl, alkoxy or phenyl groups, which are pendent or at the end of a silicone chain, these groups containing from 2 to 24 carbon atoms; phenylsilicones, for instance phenyl trimethicones, phenyl dimethicones, phenyltrimethylsiloxydiphenylsiloxanes, diphenyl dimethicones, diphenylmethyldiphenyltrisiloxanes or 2-phenylethyl trimethylsiloxy silicates, and polymethylphenylsiloxanes;
- mixtures thereof.

In the list of oils mentioned above, the term "hydrocarbon-based oil" means any oil mainly comprising carbon and hydrogen atoms, and possibly ester, ether, fluoro, carboxylic acid and/or alcohol groups.

The other fatty substances that may be present in the oily phase are, for example, fatty acids containing from 8 to 30 carbon atoms, for instance stearic acid, lauric acid, palmitic acid and oleic acid; waxes, for instance lanolin wax, beeswax, carnauba wax or candelilla wax, paraffin wax, lignite wax or microcrystalline waxes, ceresin or ozokerite, and synthetic waxes, for instance polyethylene waxes and Fischer-Tropsch waxes; silicone resins such as trifluoromethyl-C₁₋₄-alkyl dimethicone and trifluoropropyl dimethicone; and silicone elastomers, for instance the products sold under the name KSG by the company Shin-Etsu, under the name Trefil, BY29 or EPSX by the company Dow Corning, or under the name Gransil by the company Grant Industries. These fatty substances may be chosen in a varied manner by a person skilled in the art so as to prepare a composition having the desired properties, for example in terms of consistency or texture.

The proportion of the oily phase of the emulsion may range from 5% to 80% by weight and preferably from 5% to 50% by weight relative to the total weight of the composition.

The composition according to the invention may comprise an additional emulsifier other than the inulin bearing a hydrophobic carbamate or ester group described previously.

Additional emulsifiers that may be mentioned include:
oxyalkylenated (more particularly polyoxyethylenated) fatty acid esters of glycerol; oxyalkylenated fatty acid esters of sorbitan; oxyalkylenated (oxyethylenated and/or oxypropylenated) fatty acid esters; oxyalkylenated (oxyethylenated and/or oxypropylenated) fatty alcohol ethers; sugar esters such as sucrose stearate; and mixtures thereof, such as the mixture of glyceryl stearate and PEG-40 stearate;
ethoxylated (15 EO) sodium ethyldiamido-n-cocoyl sulfonate or alternatively the mixture of ethoxylated (15 EO) sodium ethyldiamido-n-cocoyl sulfonate, glyceryl stearate, glyceryl stearate monocitrate and behenyl alcohol, such as the product sold under the name Ceralution® H by the company Sasol;
dimethicone copolyols, for instance bis-PEG/PPG-16/16 PEG/PPG-16/16 dimethicone, such as the product sold under the name Abil Care 85 by the company Evonik-Goldschmidt.

According to one embodiment of the composition according to the invention, the additional emulsifier is ethoxylated (15 EO) sodium ethyldiamido-n-cocoyl sulfonate, especially the mixture of ethoxylated (15 EO) sodium ethyldiamido-n-cocoyl sulfonate, glyceryl stearate, glyceryl stearate monocitrate and behenyl alcohol, such as the product sold under the name Ceralution® H by the company Sasol.

According to one embodiment of the composition according to the invention, the additional emulsifier is a dimethicone copolyol, especially bis-PEG/PPG-16/16 PEG/PPG-16/16 dimethicone, such as the product sold under the name Abil Care 85 by the company Evonik-Goldschmidt.

The additional emulsifier may be present in the composition of the invention in a proportion ranging from 0.3% to 30% by weight and in particular from 0.5% to 20% by weight relative to the total weight of the composition.

The composition according to the invention may also contain adjuvants that are common in the field under consideration, such as emulsifiers, hydrophilic or lipophilic gelling agents, waxes, hydrophilic or lipophilic additives, preserving agents, antioxidants, solvents, fragrances, fillers, UVA and/or UVB screening agents (organic or mineral, soluble or insoluble), pigments, fibres, chelating agents, odour absorbers, dyestuffs and other cosmetic active agents.

The amounts of these various adjuvants are those conventionally used in the cosmetics field, and may range, for example, from 0.01% to 30% of the total weight of the composition. In general, the amounts are adjusted as a function of the formulation prepared. Depending on their nature, these adjuvants may be introduced into the fatty phase, into the aqueous phase and/or into lipid spherules.

Advantageously, the composition according to the invention may comprise a silicone elastomer. Examples of silicone elastomers are described in patent application WO-A-2009/080 958.

A composition according to the invention may be in the form of a care product, an antisun or after-sun product, a daily photoprotective care product, a body product, a foundation to be applied to the face or the neck, a concealer product, a complexion corrector, a tinted cream or a makeup base for making up the face, or a body makeup composition.

A composition according to the invention may be used for the purposes of improving the general condition of the epidermis, in particular of the skin, and especially for maintaining or restoring its physiological functions and/or its aesthetic appearance.

Other characteristics and advantages of the invention will emerge more clearly from the examples that follow, which are given as non-limiting illustrations. In the text hereinbelow or hereinabove, the proportions are given as weight percentages, unless otherwise indicated.

### Comparative Examples 1 to 3:

Three compositions (facial care cream) described in the table below were prepared: a composition according to the invention (Ex. 3) containing the sodium salt of 3-hydroxy-2-pentylcyclopentaneacetic acid and inulin lauryl carbamate, and two compositions not forming part of the invention: one not containing inulin lauryl carbamate (Ex. 1), the other not containing the acid active agent (Ex. 2).

The viscosity of the compositions obtained was then measured after 24 hours of storage at room temperature (viscosity measured at 25°C using a Rheomat 180 viscometer with an M3 spindle after 10 minutes of rotation at 200 rpm).
Centrifugation of the composition was also performed for 1 hour at 25°C and at 900×g.
A microscopic evaluation of the composition was also made.
The stability of each composition after storage for 2 months at 25°C was also evaluated.
The following results were obtained:

| **Example** | **1 (HI)** | **2 (HI)** | **3** |
|---|---|---|---|
| Sodium salt of 3-hydroxy-2-pentylcyclopentaneacetic acid at 30% in a water/dipropylene glycol mixture (70/30) | 13.4%, i.e. 4% AM | - | 13.4%, i.e. 4% AM |
| Water | qs 100 | qs 100 | qs 100 |
| Polyacrylamidomethylpropanesulfonic acid partially neutralized ammonia and highly crosslinked (Hostacerin AMPS® from Clariant) | 1.4 | 1.4 | 1.4 |
| Inulin lauryl carbamate (Inutec SP1 from Beneo) | - | 0.5 | 0.5 |
| Condensate of ethylene oxide and propylene oxide and ethylene oxide (128 EO/54 PO/128 EO) (Synperonic PE/F 108 from Croda) | 0.15 | 0.15 | 0.15 |
| Mixture of ethoxylated (15 EO) sodium ethyldiamido-n-cocoyl sulfonate/behenyl alcohol/glyceryl stearate/glyceryl stearate citrate (15/35/35/15) (Ceralution® H from Sasol) | 0.3 | 0.3 | 0.3 |
| Ethanol | 5 | 5 | 5 |
| Polymethyl methacrylate spheres (Covabead LH 85 from LCW) | 0.25 | 0.25 | 0.25 |
| Mixture of organopolysiloxane and cyclohexasiloxane (13/87) (Gransil RPS-D6 from Grant Industries) | 15 | 15 | 15 |
| Mixture of a,ω-dihydroxylated | 2 | 2 | 2 |
| polydimethylsiloxane/polydimethylsiloxa ne 5 cSt; Dow Corning 1503 Fluid from Dow Corning | | | |
| Polymethylsilsesquioxane (Tospearl 200 B from Momentive Performance) | 0.5 | 0.5 | 0.5 |
| Sodium hyaluronate (Cristalhyal from Soliance) | 0.2 | 0.2 | 0.2 |
| Hydroxyethylcellulose (Natrosol 250 HHR from Aqualon) | 0.15 | 0.15 | 0.15 |
| Sodium hydroxide | 0.023 | 0.023 | 0.023 |
| Silica (SB 700 from Miyoshi Kasei) | 0.5 | 0.5 | 0.5 |
| Preserving agent | qs | qs | qs |
| **viscosity (Pa.s)** | 1.07 | 2 | 1.07 |
| **centrifugation** | Non-homogeneous | Homogeneous | Remains homogeneous |
| **Microscopic appearance** | Very heterogeneous | Homogeneous | Homogeneous |
| **Stability after 2 months at 25°C** | Unstable | Stable | Stable |

These tests showed that the placebo formula with inulin lauryl carbamate (Ex. 2) is stable.
The formula containing only the sodium salt of 3-hydroxy-2-pentylcyclopentaneacetic acid (Ex. 1) is unstable: the introduction of this active agent into the placebo support destabilizes the composition.
The formula according to the invention (Ex. 3) containing the sodium salt of 3-hydroxy-2-pentylcyclopentaneacetic acid and inulin lauryl carbamate is stable. This inulin thus makes it possible to stabilize the composition containing the acid active agent.

### Example 4:

An oil-in-water emulsion facial care cream having the following composition was prepared:

| **Example** | **4** |
|---|---|
| Sodium salt of 3-hydroxy-2-pentylcyclopentaneacetic acid at 30% in a water/dipropylene glycol mixture (70/30) | 13.4%, i.e. 4% AM |
| Water | qs 100 |
| Neutralized polyacrylamidomethylpropanesulfonic acid partially neutralized ammonia and highly crosslinked (Hostacerin AMPS® from Clariant) | 1.2 |
| Inulin lauryl carbamate (Inutec SP1 from Beneo) | 0.3 |
| Sodium hyaluronate (Cristalhyal from Soliance) | 0.2 |
| Hydroxyethylcellulose (Natrosol 250 HHR from Aqualon) | 0.15 |
| Ethanol | 5 |
| Mixture of bis-PEG/PPG-16/16 PEG/PPG-16/16 dimethicone and caprylic/capric triglyceride (87/13) (Abil Care 85 from Evonik-Goldschmidt) | 2 |
| Mixture of organopolysiloxane and cyclohexasiloxane (13/87) (Gransil RPS-D6 from Grant Industries) | 15 |
| Mixture of a,ω-dihydroxylated polydimethylsiloxane/polydimethylsiloxan e 5 cSt; Dow Corning 1503 Fluid from Dow Corning | 2 |
| Pentaerythrityl tetraoctanoate | 2 |
| Polymethyl methacrylate (Micropearl M 310 from Matsumoto Yushi-Seiyaku) | 1.25 |
| Smectite (Veegum from Vanderbilt) | 2.0 |
| Preserving agent | qs |
| **viscosity (Pa.s)** | 1.2 |
| **centrifugation** | Remains homogeneous |
| **Microscopic appearance** | Homogeneous |
| **Stability after 2 months at 25°C** | Stable |

## Claims

1. Cosmetic composition in the form of an oil-in-water emulsion comprising a compound of formula (I) below: 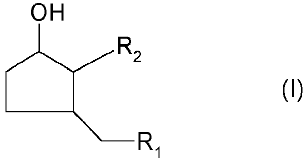 in which:
R₁ represents a radical COOR₃, R₃ denoting a hydrogen atom or a C₁-C₄ alkyl radical, optionally substituted with one or more hydroxyl groups;
- R₂ represents a saturated or unsaturated linear hydrocarbon-based radical containing from 1 to 18 carbon atoms or a saturated or unsaturated branched or cyclic hydrocarbon-based radical containing from 3 to 18 carbon atoms;
and also the optical isomers thereof, and the corresponding salts;
an inulin bearing hydrophobic groups chosen from C₄-C₃₂ alkyl carbamate groups and C₄-C₃₂ alkyl ester groups;
an oily phase and an aqueous phase.

2. Composition according to Claim 1, **characterized in that** compound (I) is such that R₁ denotes a radical chosen from -COOH, -COOMe, -COO-CH₂-CH₃, -COO-CH₂-CH(OH)-CH₂OH, -COOCH₂-CH₂-CH₂O and -COOCH₂-CH(OH)-CH₃; R₂ denotes a linear, saturated or unsaturated hydrocarbon-based radical containing from 2 to 6 carbon atoms.

3. Composition according to Claim 1 or 2, **characterized in that** compound (I) is 3-hydroxy-2-pentylcyclopentaneacetic acid.

4. Composition according to any one of the preceding claims, **characterized in that** the compound of formula (I) is present in a content ranging from 1% to 10% by weight and preferably ranging from 1.5% to 5% by weight relative to the total weight of the composition.

5. Composition according to any one of the preceding claims, **characterized in that** the hydrophobic groups of the inulin are chosen from C₁₀-C₁₈ alkyl carbamate groups and C₁₀-C₁₈ alkyl ester groups.

6. Composition according to any one of the preceding claims, **characterized in that** it comprises an inulin bearing lauryl carbamate groups.

7. Composition according to any one of the preceding claims, **characterized in that** the inulin bearing hydrophobic groups is present in a content ranging from 0.1 % to 3% by weight, preferably ranging from 0.1% to 2% by weight and preferentially ranging from 0.2% to 2.5% by weight relative to the total weight of the composition.

8. Composition according to any one of the preceding claims, **characterized in that** it comprises an additional emulsifier.

9. Non-therapeutic process for treating keratin materials, comprising the application to the said keratin materials of a cosmetic composition as defined according to one of the preceding claims.

## Patentansprüche

1. Kosmetikzusammensetzung in Form einer Öl-in-Wasser-Emulsion, umfassend eine Verbindung der Formel (I) unten: 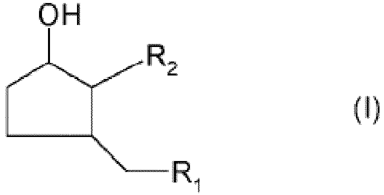 worin
R₁ einen Rest COOR₃ bedeutet, wobei R₃ ein Wasserstoffatom oder einen C₁-C₄-Alkylrest, der gegebenenfalls durch eine oder mehrere Hydroxylgruppen substituiert ist, bedeutet;
- R₂ einen gesättigten oder ungesättigten geradkettigen Rest auf Kohlenwasserstoffbasis, der 1 bis 18 Kohlenstoffatome enthält, oder einen gesättigten oder ungesättigten verzweigten oder cyclischen Rest auf Kohlenwasserstoffbasis, der 3 bis 18 Kohlenstoffatome enthält, bedeutet;
sowie die optischen Isomere davon und die entsprechenden Salze;
ein Inulin, das hydrophobe Gruppen, ausgewählt aus C₄-C₃₂-Alkylcarbamatgruppen und C₄-C₃₂-Alkylestergruppen, trägt;
eine Ölphase und eine wässrige Phase.

2. Zusammensetzung nach Anspruch 1, **dadurch gekennzeichnet, dass** die Verbindung (I) derart ist, dass R₁ einen Rest, ausgewählt aus -COOH, -COOMe, -COO-CH₂-CH₃, -COO-CH₂-CH (OH)-CH₂OH, -COOCH₂-CH₂-CH₂OH und -COOCH₂-CH(OH)-CH₃, bedeutet; R₂ einen geradkettigen, gesättigten oder ungesättigten Rest auf Kohlenwasserstoffbasis, der 2 bis 6 Kohlenstoffatome enthält, bedeutet.

3. Zusammensetzung nach Anspruch 1 oder 2, **dadurch gekennzeichnet, dass** es sich bei Verbindung (I) um 3-Hydroxy-2-pentylcyclopentanessigsäure handelt.

4. Zusammensetzung nach einem der vorhergehenden Ansprüche, **dadurch gekennzeichnet, dass** die Verbindung der Formel (I) in einem Gehalt von 1 Gew.-% bis 10 Gew.-% und vorzugsweise im Bereich von 1,5 Gew.-% bis 5 Gew.-% in Bezug auf das Gesamtgewicht der Zusammensetzung vorliegt.

5. Zusammensetzung nach einem der vorhergehenden Ansprüche, **dadurch gekennzeichnet, dass** die hydrophoben Gruppen des Inulins aus C₁₀-C₁₈-Alkylcarbamatgruppen und C₁₀-C₁₈-Alkylestergruppen ausgewählt sind.

6. Zusammensetzung nach einem der vorhergehenden Ansprüche, **dadurch gekennzeichnet, dass** sie ein Inulin, das Laurylcarbamatgruppen trägt, umfasst.

7. Zusammensetzung nach einem der vorhergehenden Ansprüche, **dadurch gekennzeichnet, dass** das Inulin, das hydrophobe Gruppen trägt, in einem Gehalt im Bereich von 0,1 Gew.-% bis 3 Gew.-%, vorzugsweise im Bereich von 0,1 Gew.-% bis 2 Gew.-% und bevorzugt im Bereich von 0,2 Gew.-% bis 2,5 Gew.-%, in Bezug auf das Gesamtgewicht der Zusammensetzung vorliegt.

8. Zusammensetzung nach einem der vorhergehenden Ansprüche, **dadurch gekennzeichnet, dass** sie einen zusätzlichen Emulgator umfasst.

9. Nichttherapeutisches Verfahren zur Behandlung von Keratinsubstanzen, bei dem man eine Kosmetikzusammensetzung wie in einem der vorhergehenden Ansprüche definiert auf die Keratinsubstanzen aufbringt.

## Revendications

1. Composition cosmétique sous forme d'émulsion huile-dans-eau comprenant un composé de formule (I) suivante : 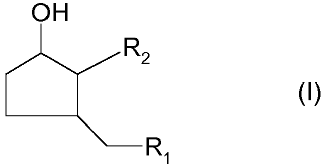 dans laquelle :
R₁ représente un radical COOR₃, R₃ désignant un atome d'hydrogène ou un radical alkyle en C₁-C₄, éventuellement substitué par un ou plusieurs groupes hydroxyle ;
R₂ représente un radical hydrocarboné, saturé ou insaturé, linéaire ayant de 1 à 18 atomes de carbone, ou ramifié ou cyclique ayant de 3 à 18 atomes de carbone ;
ainsi que leurs isomères optiques, et sels correspondants ;
une inuline à groupes hydrophobes choisis parmi les groupes alkyl C₄-C₃₂ carbamate ou alkyl C₄-C₃₂ ester; une phase huileuse et une phase aqueuse.

2. Composition selon la revendication 1, **caractérisée en ce que** le composé (I) est tel que R₁ désigne un radical choisi parmi -COOH, -COOMe, -COO-CH₂-CH₃, -COO-CH₂-CH(OH)-CH₂OH, -COOCH₂-CH₂-CH₂OH , -COOCH₂-CH (OH)-CH₃ ;
R₂ désigne un radical hydrocarboné, linéaire, saturé ou insaturé, ayant de 2 à 6 atomes de carbone.

3. Composition selon la revendication 1 ou 2, **caractérisée en ce que** le composé (I) est l'acide 3-hydroxy-2-pentylcyclopentane acétique.

4. Composition selon l'une quelconque des revendications précédentes, **caractérisée en ce que** le composé de formule (I) est présent en une teneur allant de 1 % à 10 % en poids, par rapport au poids total de la composition, de préférence de 1,5 % à 5 % en poids.

5. Composition selon l'une quelconque des revendications précédentes, **caractérisée en ce que** les groupes hydrophobes de l'inuline sont choisis parmi les groupes alkyl C₁₀-C₁₈ carbamate ou alkyl C₁₀-C₁₈ ester.

6. Composition selon l'une quelconque des revendications précédentes, **caractérisée en ce qu'**elle comprend une inuline à groupes lauryl carbamate.

7. Composition selon l'une quelconque des revendications précédentes, **caractérisée en ce que** l'inuline à groupes hydrophobes est présente en une teneur allant de 0,1 % à 3 % en poids, par rapport au poids total de la composition, de préférence allant de 0,1 % à 2 % en poids, et préférentiellement allant de 0,2 % à 2,5 % en poids.

8. Composition selon l'une quelconque des revendications précédentes, **caractérisée en ce qu'**elle comprend un émulsionnant additionnel.

9. Procédé de traitement non thérapeutique des matières kératiniques comprenant l'application sur lesdites matières kératiniques d'une composition cosmétique telle que définie selon l'une quelconque des revendications précédentes.
